# EUROPÄISCHE PATENTANMELDUNG

(11) **EP 2 067 855 A1**
(43) Veröffentlichungstag der Anmeldung: **10.06.2009**
(21) Anmeldenummer: 07023732.6
(22) Anmeldetag: 07.12.2007
(51) Int. Cl.: C12N 5/06, G01N 33/49

(54) **Anreicherung und Nachweis von fetalen Zellen aus maternalem Blut**

(71) Anmelder: Dahm, Michael W., 81677 München (DE)
(72) Erfinder: Dahm, Michael W., 81677 München (DE)
(74) Vertreter: Schüssler, Andrea

(57) **Zusammenfassung**

Die Erfindung betrifft Verfahren zur Anreicherung und zum Nachweis von fetalen Zellen aus maternalem Blut und ihre Verwendung zur pränatalen Diagnostik bzw. der Diagnose einer Schwangerschaftsgestose. Ferner betrifft die Erfindung Kits zur Durchführung des erfindungsgemäßen Verfahrens.

## Beschreibung

Die Erfindung betrifft Verfahren zur Anreicherung und zum Nachweis von fetalen Zellen aus maternalem (mütterlichem) Blut und ihre Verwendung zur pränatalen Diagnostik bzw. der Diagnose einer Schwangerschaftsgestose. Ferner betrifft die Erfindung Kits zur Durchführung des erfindungsgemäßen Verfahrens.

In den frühesten Tagen der Schwangerschaft bilden sich bereits innerhalb des Blastozystenstadiums als Produkt eines komplexen Differenzierungsprozesses zwei unterschiedliche Kompartimente aus: Das erste Kompartiment besteht aus einer inneren Zellmasse dem Embryoblast, aus dem sich der eigentliche Embryo entwickelt. Das zweite Kompartiment bildet den Trophoblast, aus dem sich ein Teil der Fruchthülle, sowie der embryonale Teil der Plazenta entwickelt. Bereits in der ersten Schwangerschaftswoche beginnen die Trophoblastzellen, an einem Pol der Blastozyste, der auch den Embryoblast enthält, in die Gebärmutterschleimhaut einzudringen. Am Ende dieser Nidation (Einnistung) steht die Plazenta, welche aus dem fetalen Trophoblast und aus der maternalen Gebärmutterschleimhaut besteht. Die Plazenta trennt wie ein passives Filterorgan den kindlichen Blutkreislauf vom mütterlichen Blutkreislauf und ermöglicht die Versorgung des Kindes mit Nährstoffen und Sauerstoff und den Abtransport von fetalen Abbauprodukten durch das Blut der Mutter.

Die Trennung von fetalem und maternalem Blut wird auch als Plazentaschranke bezeichnet. Es hat sich herausgestellt, dass die Plazentaschranke durchlässig sein kann und drei Formen von fetalen Zellen - (i) Erythroblasten, (ii) Lymphozyten und (i-ii) Trophoblastzellen in das maternale Blut gelangen und aus diesem nachgewiesen werden können (Torricelli et al. (2001) Clin Chem Lab Med 39: 494). Verschiedene Publikationen belegen die Anwesenheit von Erythroblasten und Trophoblastzellen in der maternalen Zirkulation, wobei man annehmen kann, dass deren Zahl sehr niedrig ist, da zumindest die größeren Trophoblastzellen in den maternalen Lungenkapillaren abgefangen werden. Von Holzgreve et al. wurde im Jahre 1998 ein erhöhter Transfer von fetalen Zellen in den maternalen Kreislauf bei einer Schwangerschaftsgestose (Präeklampsie) nachgewiesen (Holzgreve et al. (1998) Obstet Gynecol 91: 669).

Eine Schwangerschaftskomplikation, die unmittelbar mit der Nidation zusammenhängt wird Schwangerschaftsgestose genannt. Die Schwangerschaftsgestose ist heute immer noch eine der Hauptursachen für maternale und fetale Mortalität. Die klinischen Symptome sind insbesondere ein erhöhter Blutdruck (140/90 mmHg), Proteinurie (300g in 24 h) und Ödeme. Die Ursachen der Präeklampsie sind bis heute noch nicht ausreichend geklärt. Der pathogenetische Mechanismus liegt nach derzeitiger Meinung an einer Störung der Einnistung der Plazenta, die bedingt ist durch die Unfähigkeit der Trophoblastzellen, in die regelrechte Gebärmutterschleimhaut einzuwandern und die Arterienwände zu infiltrieren.

Wegen der großen Gefahr für Mutter und Kind, wäre in der begleitenden Schwangerschaftsdiagnostik besonders vorteilhaft, ein Nachweisverfahren zu haben, in dem Trophoblastzellen als Präeklampsiemarker im maternalen Blut nachgewiesen werden können und das als prädiktiver Test für die spätere Entwicklung einer Präeklampsie eingesetzt werden könnte.

Das Problem für ein Nachweisverfahren von fetalen Zellen, insbesondere Trophoblastzellen, besteht nun darin, dass nur sehr wenige fetale Zellen in das maternale Blut gelangen (ein E-rythroblast in 10 Millionen maternalen mononukleären Zellen, Martel-Petit et al. (2001) Prenat Diagn 21: 106) und das maternale Blut eine Vielzahl unterschiedlichster Zellen enthält. Diese Zellen bilden einen so großen zellulären Hintergrund der es unmöglich macht, die wenigen in der maternalen Blutprobe befindlichen Zielzellen nachzuweisen.

Trotz umfangreicher Forschungen ist es bislang nicht gelungen, ein einfaches, schnelles und kostengünstiges Standardverfahren zur Anreicherung von fetalen Zellen, insbesondere Trophoblastzellen, aus dem maternalen Blut zu entwickeln.

In der klinischen Erprobung wurde nun überraschend gefunden, dass diese Aufgabe durch OncoQuick^{®}, ein in WO 99/40221 und WO 00/46585 beschriebenes Verfahren zur Anreicherung von zirkulierenden Tumorzellen aus dem Blut und Knochenmark gelöst werden kann. OncoQuick^{®} (Greiner Bio One, Frickenhausen, Germany) verwendet ein Zellseparationsmedium als diskontinuierlichen Gradienten, das eine höhere Dichte als die zu untersuchende Körperflüssigkeit aufweist, und mit der Körperflüssigkeit überschichtet wird. Durch Zentrifugation werden die Zellen ihrer spezifischen Zelldichte nach aufgetrennt und können in einzelnen Fraktionen abgenommen werden (Rosenberg et al. (2002) Cytometry 49: 150). Überraschend wurde gefunden, dass durch den spezifischen Dichtegrad des Zellseparationsmediums auch fetale Zellen, insbesondere Trophoblastzellen, aus dem maternalen , insbesondere peripheren maternalen, Blut angereichert werden können.

Maternales Blut kann, wie in WO 99/40221 und WO 00/46585 beschrieben, entsprechend gängiger Standardprotokolle in einer gerinnungshemmenden Substanz abgenommen und verdünnt oder direkt unverdünnt in einem verschließbaren Zentrifugationsgefäß über das Zellseparationsmedium geschichtet werden. Als gerinnungshemmende Substanzen können beispielsweise EDTA oder Citrat bzw. Heparin oder CPD (Citrat, Phosphat, Dextrose) oder vergleichbare Substanzen eingesetzt werden. Als maternales Blut eignet sich venöses oder arterielles Blut.

Die Zentrifugation wird vorteilhaft bei ca. 500 bis 2.000 x g, bevorzugt bei ca. 1.000 x g über ca. 10 bis 30 Minuten, bevorzugt über 20-30 Minuten durchgeführt. Die Temperatur während der Zentrifugation beträgt vorzugsweise ca. 4°C.

Um einen möglichst einfachen Ablauf der Arbeiten zu sichern, kann, wie in WO 99/40221 und WO 00/46585 beschrieben, in einer bevorzugten Ausführungsform des erfindungsgemäßen Verfahrens die Zentrifugation in einem verschließbaren Gefäß durchgeführt werden, das durch eine Barriere, einen Filter oder ein Sieb, nachfolgend poröse Barriere oder Barriere genannt, in ein oberes und ein unteres Kompartiment geteilt ist, wobei das Zellseparationsmedium im unteren Kompartiment vorgelegt und die Körperflüssigkeit in das obere Kompartiment eingebracht wird.

Die Barriere hat eine poröse Beschaffenheit, die es erlaubt, dass bei der Zentrifugation Flüssigkeiten sowie die korpuskulären Bestandteile des Blutes, wie die Zellen des roten und weißen Blutsystems, welche eine höhere Dichte als das vorgelegte Zellseparationsmedium haben, die poröse Barriere ungehindert passieren können. Dies hat zur Folge, dass das Zellseparationsmedium während der Zentrifugation durch die poröse Membran in das obere Kompartiment gedrängt wird und die fetalen Zellen, insbesondere die Trophoblastzellen, welche eine geringere Dichte als das vorgelegte Zellseparationsmedium haben, auf einer Ebene oberhalb der Barriere zu liegen kommen.

In einem Zentrifugationsgefäß kann alternativ zur Barriere, wie in WO 99/40221 und WO 00/46585 beschrieben, auch eine Klappe eingesetzt werden, die das Zentrifugationsgefäß analog zur Barriere in ein oberes und ein unteres Kompartiment unterteilt.

Diese Klappe hat eine Beschaffenheit, die es erlaubt, dass sie vor und nach der Zentrifugation dicht verschlossen ist und nur während der Zentrifugation durch die Zentrifugationskraft geöffnet wird. Während der Zentrifugation treffen, bei geöffneter Klappe, die Flüssigkeiten des unteren und des oberen Kompartiments zusammen. Dies hat zur Folge, dass die Zellen des roten und weißen Blutsystems, welche eine höhere Dichte als das vorgelegte Zellseparationsmedium haben, in das untere Kompartiment gelangen und das Zellseparationsmedium in das obere Kompartiment drängen. Dies hat den Effekt, dass die fetalen Zellen, insbesondere die Trophoblastzellen, welche eine geringere Dichte als das vorgelegte Zellseparationsmedium haben, auf einer Ebene oberhalb der Klappe zu liegen kommen.

Mit Hilfe der porösen Barriere oder Klappe kann das zu untersuchende maternale Blut in das Zentrifugationsgefäß gefüllt werden, ohne dass sie sich mit dem darunterliegenden Zellseparationsmedium vermischt und somit die Anreicherung beeinträchtigen oder unmöglich machen kann.

Nach der Zentrifugation befinden sich die im maternalen Blut vorhandenen fetalen Zellen, insbesondere die Trophoblastzellen, in der Interphase des oberen Kompartiments des Zentrifugationsgefäßes. Das gesamte obere Kompartiment kann anschließend gesammelt und beispielsweise in ein frisches Zentrifugationsgefäß (vorzugsweise aus gegebenenfalls silikonisiertem Kunststoff und mit der gleichen Volumenkapazität wie das zuvor verwendete Zentrifugationsgefäß) überführt werden. Die poröse Barriere bzw. die geschlossene Klappe verhindern bei der Entnahme des verbleibenden Überstands ein Vermischen der Zellen des oberen und des unteren Kompartiments.

Vorteilhaft wird anschließend das obere Kompartiment des Zentrifugationsgefäßes beispielsweise, wie in WO 99/40221 und WO 00/46585 beschrieben, noch zweimal mit einem Puffer (z.B. PBS mit 0,1% - 10% BSA) nachgewaschen und dieser wird ebenfalls in das frische Zentrifugationsgefäß überführt. Nach einer weiteren Zentrifugation beispielsweise bei 1.000 x g über ca. 10 Minuten bei einer Temperatur von ca. 4°C können die gesammelten Zellen dann den einzelnen Nachweismethoden für fetale Zellen und insbesondere für Trophoblastzellen zugeführt werden.

In einem in WO 06/087220 beschriebenen Verfahren kann alternativ die Anreicherung der fetalen Zellen, insbesondere der Trophoblasten, aus dem maternalen Blut auch innerhalb eines evakuierten Blutabnahmegefäßes vollzogen werden. In diesem Fall ist in dem Blutabnahmegefäß statt eines Trennmediums und einer porösen Barriere bzw. Klappe, ein thixotropes Gel vorgelegt. Das thixotrope Gel ist so beschaffen, dass es die in WO 99/40221 und WO 00/46585 beschrieben Dichteeigenschaften besitzt. Während der Zentrifugation können Flüssigkeiten sowie die korpuskulären Bestandteile des Blutes, wie die Zellen des roten und weißen Blutsystems, welche eine höhere Dichte als das vorgelegte thixotrope Gel haben, das verflüssigte Gel ungehindert passieren. Dies hat zur Folge, dass die fetalen Zellen, insbesondere die Trophoblastzellen, welche eine geringere Dichte als das vorgelegte thixotrope Gel haben, nach der Zentrifugation und analog zu OncoQuick^{®} auf einer Ebene oberhalb des wieder verfestigten thixotropen Gels im oberen Kompartiment zu liegen kommen und aus diesem entnommen werden können.

Mit dem erfindungsgemäßen Verfahren können fetale Zellen, insbesondere Trophoblastzellen, aufgrund ihrer unterschiedlichen Dichte in hohem Maße von den korpuskulären Bestandteilen von Körperflüssigkeiten wie z.B. den Zellen des roten und weißen Blutsystems getrennt, und angereichert werden. Die fetalen Zellen, insbesondere Trophoblastzellen, können dann durch übliche Verfahren, z.B. über eine spezifische Antigenexpression der angereicherten fetalen Zellen, insbesondere der Trophoblastzellen, mittels immunzytologischer bzw. über eine spezifische Genexpression der angereicherten fetalen Zellen, insbesondere der Trophoblastzellen, mittels molekularbiologischer Nachweisverfahren nachgewiesen werden.

Die Verfahren zum Nachweis von fetalen Zellen, insbesondere von Trophoblastzellen, umfassen das gesamte Spektrum der gängigen Diagnostik. Beispiele hierfür sind die mikroskopischen, immunzytologischen/immunzytochemischen, biochemischen und/oder molekularbiologischen Verfahren. Beispielsweise können die fetalen Zellen, insbesondere die Trophoblastzellen, nach der Anreicherung als ganze Zellen oder als Zellenbestandteile direkt oder nach Zellkultur und Expansion der fetalen Zellen, insbesondere der Trophoblastzellen, durch morphologische, immunzytologische/immunzytochemische, biochemische und/oder molekularbiologische Verfahren nachgewiesen werden. Diese Verfahren ermöglichen den Nachweis einer ganzen Zelle, der spezifischen Aktivität einer Zelle oder den Nachweis von spezifischen Bestandteilen einer ganzen Zelle. Beispiele von Bestandteilen einer ganzen Zelle sind u.a. Proteine und Glykoproteine der Membran, des Zytoplasmas oder des Zellkerns, sowie die Chromosomen, spezifische Abschnitte von Chromosomen bis hin zu Nukleinsäuresequenzen, wie DNA, RNA und cDNA.

Beispiele direkter Zellnachweisverfahren sind u.a. sämtliche Formen der Mikroskopie, einschließlich der Färbung von Zellen oder Zellbestandteilen. Beispiele direkter Färbung sind die Trypan-Blau-Färbung oder die Färbung durch spezifische Antikörper, die gegen spezifische Bestandteile der Zelle, wie der Zellmembran, das Zytoplasma oder der Zellkern gerichtet sind und an denen Markierungssignale wie z.B. fluoreszierende Farbstoffe gekoppelt sind. Nachweisverfahren sind u.a. Durchflußzytometrie oder FACS (Fluorescence activated cell sorting), ELISA und Western Blotting. Weitere Verfahren zum Nachweis von Zellbestandteilen sind u.a. die Detektionsverfahren von Nukleinsäuren mit Hilfe von markierten Sonden, z. B. FISH, in situ Hybridisierung, Northern, South-Western und Southern Blotting oder differential display sowie u.a. die Amplifikationsverfahren von Nukleinsäuren u.a. die PCR, RT-PCR, in situ RT-PCR, Real-Time PCT und NASBA, sowie Gene Arrays für den Nachweis von zellspezifischen DNAs oder RNAs.

Beispiele für den zellulären Nachweis von Erythroblasten sind der (i) Kleihauer Betke-Test (Kleihauer et al. (1957) Klein Wochenschr 35: 637, Betke et al. (1967) Nature 214: 188) und die Verwendung von Antikörpern, die gegen (ii) den Transferrinrezeptor (CD71, Troeger et al. (1999) Prenat Diagn 19: 521), (iii) gegen Glycophorin A (GPA, Troeger et al. (1999) Prenat Diagn 19: 521), (iv) gegen das i-Blutgruppenantigen (Troeger et al. (1999) Prenat Diagn 19: 521), (v) gegen das human erythroid cell surface antigen (HAE9, Savion et al. (1997) Biol Neonate 71: 126) und (vi) gegen das fetale Hämoglobin (HbF, Oosterwijk et al. (1998) Cytometry 32: 170).

Zu den Charakteristika von Trophoblastzellen gehören die Expression bestimmter HLA-Klasse I Antigene (HLA-C, E, und G), von denen z.B. das lösliche HLA-G für die Suppression der maternalen Immunantwort angesehen wird. Beispiele für den zellulären Nachweis von Trophoblastzellen sind daher die Verwendung von spezifischen Antikörpern gegen Antigene der HLA-Klasse I, insbesondere gegen HLA-C, E, und G. Weitere Beispiele sind Antikörper gegen die Glukose-Carrierproteine GLUT1 und GLUT3.

Eine besondere Verwendung im Sinne des erfinderischen Verfahrens zum Nachweis von Trophoblastzellen sind Antikörper, die gegen Zytokeratine gerichtet sind, insbesondere der monoklonale Zytokeratin-Antikörper A45 B/B3 (Muller et al (2005) Clin Cancer Res 111: 3678), bzw. der molekulare Nachweis der Zytokeratin Genexpression. Zytokeratine gehören zu der Familie von filamentösen Proteinen, die am Aufbau des Zytoskeletts von e-pithelialen Zellen beteiligt sind (Moll et al. in Cell (1982) 31: 11). Nachdem epitheliale Zellen im Blut normalerweise nicht vorkommen, wird im Sinne des erfinderischen Verfahrens postuliert, dass eine Zytokeratinexpression im Blut ein Surrogatmarker für das Vorhandensein von fetalen Trophoblastzellen im maternalen Blut ist.

Wie in WO 04/023110 beschrieben, können auch andere Blutzellen wie polymorphkernige Leukozyten, speziell Granulozyten, Zytokeratine exprimieren (Jung et al. Br J Cancer (1999) 81: 870). Aufgrund dieser Tatsache muss vor dem Nachweis einer Zytokeratinexpression als Surrogatmarker für Trophoblastzellen im Blut eine Trennung der Zytokeratin-exprimierenden nicht-Trophoblastzellen von den Zytokeratin-exprimierenden Trophoblastzellen vorgenommen werden. Es wurde überraschend gefunden, dass diese Aufgabe durch OncoQuick^{®} gelöst werden kann, da durch den spezifischen Dichtegrad des Zellseparationsmediums Zytokeratin-positive von Zytokeratin-negativen hämatopoetischen Zellen getrennt werden können. Das Ergebnis der Zentrifugation mit OncoQuick^{®} ist, dass sich die angereicherten Trophoblastzellen nach der Zentrifugation in der gleichen Fraktion befinden wie die Zytokeratin-negativen hämatopoetischen Zellen, so dass eine anschließend nachgewiesene molekulare Zytokeratinexpression in dieser Fraktion zweifelsfrei auf vorhandene Trophoblastzellen zurückgeführt werden kann.

Das erfindungsgemäße Verfahren eignet sich zur pränatalen Diagnostik bzw. zur Diagnose einer Schwangerschaftsgestose.

Der Nachweis der Trophoblastzellen kann direkt als Gestosemarker eingesetzt werden. Einen besonderen Stellenwert wird dem Verfahren im Rahmen der begleitenden Schwangerschaftsdiagnostik beigemessen, da mit einer erheblich früheren Diagnose und bei sofortiger Therapie mit einer sichereren Schwangerschaft zu rechnen ist.

Die vorliegende Erfindung betrifft somit auch ein Verfahren zum Nachweis von fetalen Zellen, insbesondere von Trophoblastzellen, in maternalem Blut, worin die fetalen Zellen, insbesondere die Trophoblastzellen, wie vorstehend beschrieben, aus dem maternalen Blut angereichert und bevorzugt gleichzeitig unerwünschte Zellen abgereichert und die fetalen Zellen, insbesondere die Trophoblastzellen, mittels immunzytologischer oder molekularbiologischer Techniken nachgewiesen werden.

Ein weiterer Gegenstand der vorliegenden Erfindung ist ein Kit zur Anreicherung von fetalen Zellen, insbesondere Trophoblastzellen, aus dem maternalen Blut, der zur Durchführung des erfindungsgemäßen Verfahrens geeignet ist. Hierzu umfasst der Kit das in WO 99/40221 und WO 00/46585 offenbarte Zellseparationsmedium, sowie gegebenenfalls ein Zentrifugationsgefäß, bzw. das in WO 06/087220 offenbarte evakuierte Blutabnahmegefäß mit einem thixotropen Gel, das den Spezifikationen in WO 99/40221 und WO 00/46585 entspricht.

Ein weiterer Gegenstand der vorliegenden Erfindung ist ein immunzytologischer Kit zum Nachweis der aus dem maternalen Blut angereicherten fetalen Zellen, insbesondere Trophoblastzellen, der zur Durchführung des erfindungsgemäßen Verfahrens geeignet ist. Hierzu umfasst der Kit spezifische monoklonale Antikörper, mit denen fetale Zellen, insbesondere Trophoblastzellen, immunzytologisch nachgewiesen werden können. Eine besondere Form des Kits ist die Kombination mit dem monoklonalen Zytokeratin-Antikörper A45 B/B3 (Muller et al (2005) Clin Cancer Res 111: 3678).

Ein weiterer Gegenstand der vorliegenden Erfindung ist ein molekularbiologischer Kit zum Nachweis der aus dem maternalen Blut angereicherten fetalen Zellen, insbesondere Trophoblastzellen, der zur Durchführung des erfindungsgemäßen Verfahrens geeignet ist. Hierzu umfasst der Kit spezifische Primer und Enzyme, mit denen spezifische Gene von fetalen Zellen, insbesondere Trophoblastzellen, mittels Reverse Transkriptase Polymerase Kettenreaktion (RT-PCR) oder mittels Real Time- Polymerase Kettenreaktion (PCR) nachgewiesen werden können. Eine weitere Variante zu einem moleklarbiologischen Kit ist ein Gene Array der spezifische Gene von fetalen Zellen, insbesondere Trophoblastzellen, enthält.

### Beispiel 1: Nachweis von Trophoblastzellen aus peripherem Blut

Von insgesamt 50 Patientinnen mit einer Schwangerschaftsgestose wurde jeweils eine Gesamtmenge von 20 ml peripheren Blutes abgenommen und mit OncoQuick^{®} (Greiner Bio One) nach Angaben des Herstellers aufgereinigt. Das gesamte obere Kompartiment wurde entnommen und in ein frisches 50 ml Zentrifugationsgefäß überführt. Die gesammelten Zellen wurden einmal mit dem Onco-Quick^{®}-Waschpuffer bei 200g für 12 Minuten bei 4°C gewaschen. Die gewaschenen Zellen wurden dann gesammelt und mittels Zytospin auf Objektträger zentrifugiert und mit dem monoklonalen Zytokeratin-Antikörper A45 B/B3 (Muller et al. (2005) Clin Cancer Res 111: 3678) inkubiert. Das Ergebnis dieser Untersuchungen zeigte, dass in über 50% der Patientinnen Trophoblastzellen nachgewiesen werden konnten. In weiteren Untersuchungen von peripherem Blut einer Kontrollgruppe von nicht schwangeren Frauen wurden keine Trophoblastzellen gefunden.

### Beispiel 2: Nachweis von fetalen Erythroblasten aus Nabelschnurblut

Von insgesamt 30 Proben von Nabelschnurblut wurde jeweils eine Gesamtmenge von 20 ml Nabelschnurblut mit OncoQuick^{®} (Greiner Bio One) nach Angaben des Herstellers aufgereinigt. Das gesamte obere Kompartiment wurde entnommen und in ein frisches 50 ml Zentrifugationsgefäß überführt. Die gesammelten Zellen wurden einmal mit dem OncoQuick^{®}-Waschpuffer bei 200g für 12 Minuten bei 4°C gewaschen. Die gewaschenen Zellen wurden dann gesammelt und mittels Zytospin auf Objektträger zentrifugiert und mit monoklonalen Antikörper CD71 inkubiert. Das Ergebnis dieser Untersuchungen zeigte, dass in über 90% der Fälle fetale Erythroblasten nachgewiesen werden konnten. In einer Vergleichsuntersuchung, die wie vorstehend beschrieben an Nabelschnurblut durchgeführt wurde, konnten nach einer Inkubation mit einem monoklonalen Antikörper gegen fetales Hämoglobin keine Erythroblasten nachgewiesen werden.

## Patentansprüche

1. Verfahren zur Anreicherung von fetalen Zellen aus maternalem Blut, bei dem ein Zellseparationsmedium mit einer Dichte von 1.055 bis 1.065g/ml mit dem Blut überschichtet und zentrifugiert wird, wodurch die fetalen Zellen von den korpuskulären Bestandteilen des Blutes abgetrennt werden.

2. Verfahren nach Anspruch 1, wobei das Blut peripheres Blut ist.

3. Verfahren nach Anspruch 1 oder 2, wobei die fetalen Zellen Trophoblastzellen sind.

4. Verfahren nach einen der Ansprüche 1-3, bei dem die fetalen Zellen durch mikroskopische, immunzytologische, immunzytochemische, biochemische und/oder molekularbiologische Verfahren nachgewiesen werden.

5. Verwendung der Verfahren nach einem der Ansprüche 1-4 zur pränatalen Diagnostik bzw. zur Diagnose einer Schwangerschaftsgestose.

6. Kit zur Durchführung der Verfahren nach einem der Ansprüche 1-5, umfassend ein Zellseparationsmedium mit einer Dichte von 1.055 bis 1.065 g/ml.

7. Kit nach Anspruch 6, umfassend ein Mittel zum Nachweis von fetalen Zellen.
